# EUROPEAN PATENT SPECIFICATION

(11) **EP 4 515 293 B1**
(45) Date of publication and mention of the grant of the patent: **04.11.2026**
(21) Application number: 23721458.0
(22) Date of filing: 24.04.2023
(51) Int. Cl.: G01T 1/36, G01N 23/00, G06N 3/00

(54) **SYSTEM AND METHOD FOR IDENTIFYING ISOTOPES**
SYSTEM UND VERFAHREN ZUR IDENTIFIZIERUNG VON ISOTOPEN
SYSTÈME ET PROCÉDÉ D'IDENTIFICATION D'ISOTOPES

(30) Priority: 25.04.2022 GB 202205955
(43) Date of publication of application: 05.03.2025
(73) Proprietor: Kromek Limited, Sedgefield, Durham TS21 3FD (GB)
(72) Inventor: ZHANG, Siru, Liverpool Liverpool Merseyside L69 3BX (GB); GOULERMAS, Yannis, Liverpool Liverpool Merseyside L69 3BX (GB); MARSDEN, Edward, Durham TS21 3FD (GB)
(74) Representative: Murgitroyd & Company
(86) International application number: PCT/GB2023/051084
(87) International publication number: WO 2023/209352

(56) References cited:
- WO-A1-2021/026280
- WO-A1-2022/031316
- IT-A1- 202000 025 006
- US-A1- 2013 054 603
- ELY J ET AL: "The use of energy windowing to discriminate SNM from NORM in radiation portal monitors", NUCLEAR INSTRUMENTS & METHODS IN PHYSICS RESEARCH. SECTION A, ELSEVIER BV * NORTH-HOLLAND, NL, vol. 560, no. 2, 10 May 2006 (2006-05-10), pages 373 - 387, XP027924254, ISSN: 0168-9002, [retrieved on 20060510]

## Description

### TECHNICAL FIELD

The present invention relates to a method for identifying isotopes. In particular, the present invention relates to a computer-implemented method for determining the presence of a radiological source.

### BACKGROUND

In the past decade, there has been an increasing need to screen for nuclear or radioactive sources, and to prevent threats to the security of the general public. The deployment of inspection systems and portable monitoring services, employing passive gamma and neutron radiation detectors, provides a useful layer of defence towards public safety. However, an aim to improve such detectors in their detection efficiency and accuracy is required to meet the conflicting demands of having a high sensitivity to detection of weak radioactive sources, whilst minimising susceptibility of systems to false alarms, which may be time consuming and costly.

There are several problems faced by developers in the radiation detector industry. Firstly, environmental noise and signal attenuation may impede the detection accuracy. For example, when a detector operates in a city street, walls and materials present in buildings heavily attenuate radiation and therefore affect the ability for the detector to identify the radioactive source. Moreover, this issue must be compensated for in real-time for live detection. Other factors, such as calibration shift, the presence of masking sources including naturally occurring radiation materials (NORM) and medical isotopes as well as widely varying source strength complicate and can hinder detection even further.

Various methods have been used to detect and identify radioactive sources. The simplest way is to consider only the gross counts of gamma rays from the radioactive source by ignoring the spectrum information, and to alert a user when a total count exceeds a count threshold. However, more sophisticated approaches exist, such as some machine learning algorithms, which can incorporate and use full radiation spectral information. Most recent machine learning algorithm approaches work exclusively using spectral data simulated in, for example, M. Kamuda and C. J. Sullivan, "An automated isotope identification and quantification algorithm for isotope mixtures in low-resolution gamma-ray spectra," Radiation Physics and Chemistry, vol. 155, pp. 281-286, 2019 as the input to the detection algorithms. However, these spectra cannot entirely reproduce the complicated characteristics that appear in real-world situations and this may lead to low detection accuracies in a deployed system using these approaches. Document WO 2022/031316 A1 discloses an isotope identification method utilising a single artificial neural network (ANN).

The present invention has been devised to mitigate or overcome at least some of the above-mentioned problems.

### SUMMARY OF THE INVENTION

In accordance with a first aspect of the present invention, there is provided an isotope identification method performed by a computing device, according to claim 1.

An example of a spectroscopy device useful for understanding, but not forming part of, the presently claimed invention may be any device suitable for collecting real-world isotope spectra. For example, the spectroscopy device may be a scintillation counter configured to detect and measure the intensity and/or energy of ionizing radiation emitted by isotopes and incident upon the scintillation counter. Optionally, the spectral data may be filtered to match a characteristic resolution of the spectroscopy device, in order to reduce artificial features generated by statistical noise.

The skilled addressee will understand that the term "isotope probability" means a probability that a dataset corresponds to a particular isotope, for example ¹³⁷Cs.

Gamma rays with different energies emitted by isotopes during nuclear decay are detected by the spectroscopy device. The spectroscopy device provides the plurality of spectral data to the computing device, wherein the spectral data may be the gamma ray energy detected by the spectroscopy device. A machine-learning based approach is employed to classify or identify the isotopes associated with the gamma rays. The present invention therefore provides a method for identifying and classifying individual isotopes. The present method also finds use in multi-label cases, wherein an isotope mixture comprising a plurality of isotopes is to be classified. The method advantageously has robustness to noise and classifies isotopes with an increased accuracy.

Preferably, each dataset of the plurality of datasets comprises binned data, said dataset being representative of a functional relationship between a first bin of the binned data and a second bin of the binned data. In particular, the spectral data collected from the spectroscopy device is grouped according to a plurality of intervals (i.e. bins), and a value is assigned to each interval. The bins may each be, for example, a particular energy range of the gamma rays received at the spectroscopy device. An example bin interval is 10³ to 10⁴ eV. A bin count is associated with each bin, wherein the bin count is indicative of the number of gamma rays the spectroscopy device has captured within the bin interval.

In preferable embodiments, the plurality of datasets each comprise a respective bin-ratio vector. The skilled addressee will understand the term "bin-ratio vector" to be a vector in which each entry is a direct ratio between a pair of bin counts of different bins. Advantageously, use of bin-ratio vectors increases classification accuracy, particularly for examples wherein there is a large energy range of gamma rays detected. In particular, different spectra collected for the same isotopes may have bins having significantly different values. The difference in values may be due to variations in radiation source strength, location or background rate. The measure of similarity between spectra may be affected by this partial matching. The bin-ratio vector may captures the relative size of peaks in spectra, which may in turn improve the robustness of the present method.

In some embodiments, generating the plurality of datasets comprises: binning, by the processor, the plurality of spectral data into n spectral bins; generating, by the processor, a plurality of bin-ratio vectors; and storing, by the processor, in each dataset of the plurality of datasets, a respective bin-ratio vector.

Preferably, generating the plurality of bin-ratio vectors comprises: generating, by the processor, a histogram based on the n spectral bins; generating, by the processor, a histogram ratio matrix M based on the histogram; wherein the histogram ratio matrix M is a square matrix of length n; and extracting, by the processor, the plurality of bin-ratio vectors from the histogram ratio matrix; wherein there are k bin-ratio vectors and k = n-1. In particular, the histogram ratio matrix M is a square matrix of length n, in which each element is a ratio between a pair of bin counts. The histogram ratio matrix M will be understood by the skilled addressee as meaning a bin-ratio matrix in which each element is the direct ratio of between a pair of bin counts.

Preferably, each entry M_{i,j} of the histogram ratio matrix M is the i^{th} bin divided by the j^{th} bin. Accordingly, a ratio between bin counts is represented in the histogram ratio matrix m.

Preferably, the entries of the y^{th} bin-ratio vector are a bin-ratio of a first bin and a second bin, wherein the second bin is separated from the first bin by y bins.

Accordingly, each bin-ratio vector comprises entries that are a distinct ratio of bin counts.

In alternative embodiments, the bin data comprises a bin difference. That is, the bin data comprises a difference between a first bin count and a second bin count.

In the invention according to the wording of the claims, the isotope probability is determined by using an ensemble; said ensemble comprising a plurality of classifications generated by a multiclass classifier, each classification corresponding to a respective dataset. The term "multiclass classifier" will be understood by the skilled addressee as meaning a classification model configured to classify the datasets into one of the plurality of classifications. The classifications are indicative of an isotope. The term "ensemble" will be understood by the skilled addressee as meaning a collection of classification models. The multiclass classifiers may be one or more selected from, but not limited to: a k-nearest neighbours (k-NN) classification method; a logistic regression (LR) classification method; a linear discriminant analysis (LDA) classification method; a support vector machine (SVM) classification method; an artificial neural networks (ANN) classification method; and a convolution neural network (CNN) classification method. The skilled addressee will appreciate that any combination of classification methods may be used. Further, the skilled addressee will understand that since the functions for k-NN, LR, LDA, and SVM only support binary classification, an additional binary relevance method is applied to the multi-label cases in order to transform them into multiple separate binary problems.

In the invention according to the wording of the claims, the ensemble is an artificial neural network (ANN) ensemble; said ANN ensemble comprising a plurality of ANN classifications, each generated by an ANN, and each ANN classification corresponding to a respective dataset. The skilled addressee will understand that the term "ANN classification" means an isotope classification or identification determined by the ANN. Accordingly, using the ANN ensemble, multiple ANNs are trained rather than a single ANN. Advantageously, the present invention provides improved generalisation ability. Further advantageously, a variance of predictions is reduced using the present invention.

In some embodiments, the isotope probability is determined based on the plurality of ANN classifications.

Preferably, each ANN comprises one or more parameters, the parameters being one or more selected from the range of: a hidden layer comprising a number of neurons; a learning rate; and a regularization strength.

Preferably, the parameters are selected by applying a random search method.

Preferably, the multiclass classifier is optimised.

In some embodiments, the multiclass classifier is optimised through error backpropagation, the multiclass classifier being configured to minimize a cost function with respect to a network weight.

Preferably, the cost function is a cross-entropy loss function for single isotope classification.

In some embodiments, the cost function is a mean-squared error cost function for isotope mixture classification.

In some embodiments, the cost function comprises a penalty term, said penalty term being an L2 regularization element.

In alternative embodiments, the multiclass classifier is optimised according to a scaled conjugate gradient method.

Preferably, the multiclass classifier is trained until a threshold difference between a predicted output and a correct output reaches a threshold.

Preferably, the isotope is identified by an ensemble forecast.

In some embodiments, the ensemble forecast comprises: determining the isotope having the highest probability for each dataset of the plurality of datasets; and identifying the isotope having the highest number of highest probabilities.

It will be appreciated that any features described herein as being suitable for incorporation into one or more aspects or embodiments of the present disclosure are intended to be generalizable across any and all aspects and embodiments of the present disclosure. Other aspects of the present disclosure can be understood by those skilled in the art in light of the description, the claims, and the drawings of the present disclosure. The foregoing general description and the following detailed description are exemplary and explanatory only and are not restrictive of the claims.

### BRIEF DESCRIPTION OF THE DRAWINGS

One or more embodiments of the invention will now be described, by way of example only, with reference to the accompanying drawings, in which:
Figure 1 is a system for identifying isotopes;
Figure 2 is a method for identifying isotopes using the system of Figure 1;
Figure 3 is a histogram ratio matrix illustrating the extraction of bin-ratio vectors;
Figure 4 is a schematic diagram of an artificial neural network architecture; and
Figure 5 is a method for training an artificial neural network model.

### DETAILED DESCRIPTION

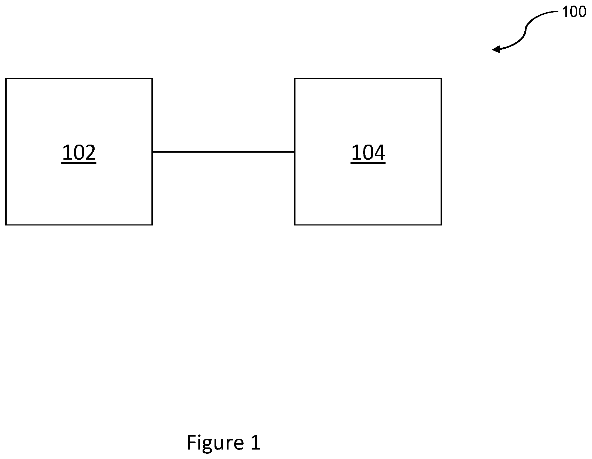

Figure 1 is a system 100 for identifying isotopes. The system 100 comprises a computing device 102 in communication with a spectrometer 104.

The spectrometer 104 is configured to receive gamma radiation from an isotope source and measure spectral components (i.e. spectral data) of the isotope source. In the present example, the spectrometer 104 is a scintillation counter 104 configured to detect and measure the intensity and/or energy of ionizing radiation emitted by isotopes and incident upon the scintillation counter. For example, the scintillation counter 104 may measure energy indicative of the isotope being any one or more of, but not limited to, the isotopes: ⁵⁷Co ⁶⁰Co; ⁶⁷Ga; and ¹³¹I. The skilled addressee will understand that this isotope list is for illustrative purposes only, and that the scintillation counter 104 may measure energies indicative of any known isotope.

The computing device 102 is configured to receive spectral data from the spectrometer 104 and perform an artificial neural network (ANN) ensemble model generation method 200, and an isotope identification method 500 in order to identify the isotope.

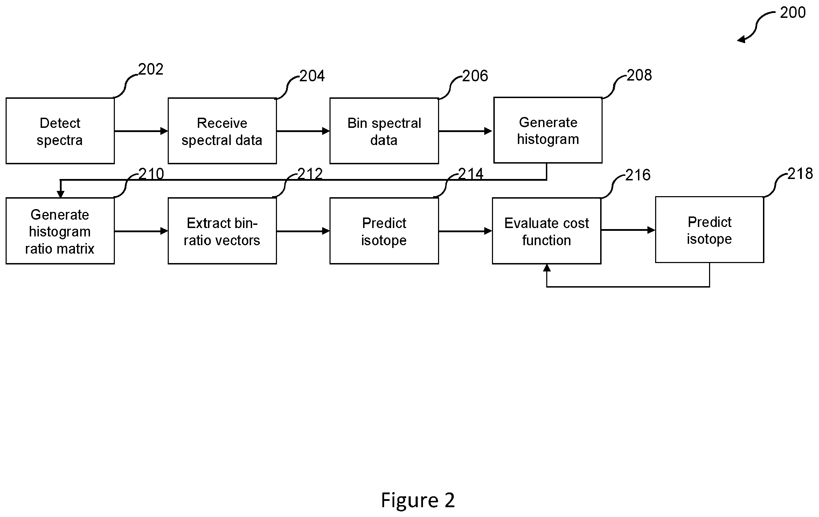

Prior to the execution of the isotope identification method 500, an artificial neural network (ANN) ensemble model is generated or pre-trained according to the training method 200 depicted in Figure 2, using the isotope identification system 100.

In a first step 202, the spectrometer detects spectra from gamma rays emitted by a plurality of known isotopes over a time period, such that a spectra count can be determined. The skilled addressee will understand the term "known isotopes" to mean isotopes that are known to the user. There may be any number of known isotopes used for this training process, such as 18 isotopes.

At step 204, the computing device 102 collects or receives training spectral data for each of the plurality of known isotopes from the spectrometer 104. The training spectral data corresponds to the detected spectra of the gamma rays received from the known isotopes. The spectral data represents energy associated with the gamma rays received and measured by the spectrometer 104. Accordingly, a gamma ray spectrum is generated.

Optionally, the spectral data may be filtered to match a characteristic resolution of the spectrometer 104, in order to reduce artificial features generated by statistical noise. Further optionally, randomly generated gain shift noise may be added to the spectral data in order to simulate a realistic setting. The gain shift noise maybe added by introducing a small random change in a linear term of a quadratic calibration curve of the spectrometer 104. Further optionally, a random background noise pattern may be added to the spectral data. A plurality of different randomly generated background noise patterns may be mixed in random proportions in order to generate multiple background spectra.

At step 206, the training spectral data is binned into n spectral bins for each of the plurality of known isotopes. That is, each of the training spectral data are grouped according to their energy. For example, for n = 1024, 1024 bins may be selected, each corresponding to a particular energy range. For a measured energy range of 0 to 10⁷ eV, each bin may span a range of around 10³ eV. More preferably, for a measured energy range of 0 to 3 x 10⁶ eV, each bin span a range of 2.93 x 10³ eV. Accordingly, each of the spectral data are allocated to one of the 1024 bins corresponding to the measured energy.

At step 208, a training histogram is generated for each of the plurality of known isotopes based on the n spectral bins. The training histogram comprises n entries and provides an indication of how many of the training spectral data fall within each bin. In other words, the training histogram provides a bin count associated with each bin. In the present example, the training histogram comprises 1024 entries.

At step 210, a training histogram ratio matrix M is generated for each of the plurality of known isotopes. The training histogram ratio matrix M is a square matrix of length n. In the present example, the training histogram ratio matrix M is a square matrix of length 1024. The entries M_{i,j} of the training histogram ratio matrix M are the bin count of the i^{th} bin divided by the bin count of the j^{th} bin: $M = {\left[\frac{{h}_{i}}{{h}_{j}}\right]}_{1 \leq i , j \leq n} = \left[\begin{matrix}\frac{{h}_{1}}{{h}_{1}} & \frac{{h}_{2}}{{h}_{1}} & ⋯ & \frac{{h}_{n}}{{h}_{1}} \\ \frac{{h}_{1}}{{h}_{2}} & \frac{{h}_{2}}{{h}_{2}} & ⋯ & \frac{{h}_{n}}{{h}_{2}} \\ ⋮ & ⋮ & ⋱ & ⋮ \\ \frac{{h}_{1}}{{h}_{n}} & \frac{{h}_{2}}{{h}_{n}} & … & \frac{{h}_{n}}{{h}_{n}}\end{matrix}\right]$

The skilled addressee will appreciate that the ratios are reciprocal and as such, the upper and lower triangles of the matrix are identical and the matrix diagonal is constant.

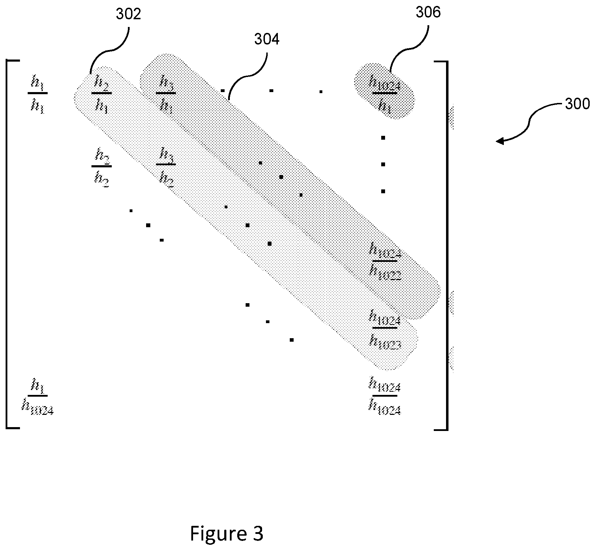

At step 212, a plurality of training bin-ratio vectors are extracted from each of the training histogram ratio matrices M. This extraction step can be more easily understood with reference to Figure 3, which depicts a training histogram ratio matrix 300 of a 1024 bin histogram.

For a training histogram ratio matrix M of n bins, the number of bin-ratio vectors is n-1. Accordingly, the training histogram ratio matrix M of the present example having 1024 bins, has 1023 bin-ratio vectors. The entries of the yth training bin-ratio vector are a bin-ratio of a first bin and a second bin, wherein the second bin is separated from the first bin by y bins. In particular, a first training bin-ratio vector 302 (having a length of 1023) contains ratios between the bin counts of adjacent bins (i.e. separated by 1 bin). A second training bin-ratio vector 304 (having a length of 1022) contains ratios between bin counts of second neighbours. A final (i.e. the 1023rd) training bin-ratio vector 306 contains a ratio between the last (i.e. 1024th bin count) and the first bin count, due to the separation of 1023 bins. Advantageously, feature diversity is introduced through ratios measured at multiple distance scales between the bins, thereby improving the robustness of the present method.

The bin-ratio vectors may each be stored in a respective dataset.

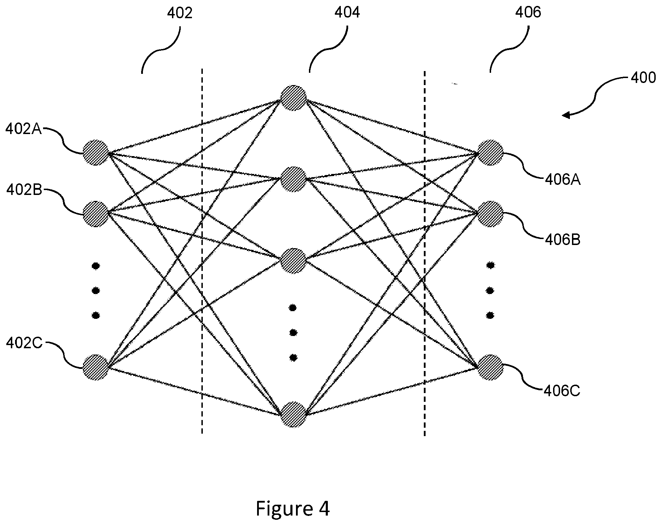

At step 214, a first set of isotope predictions is determined for the first bin-ratio vector 302 of each of the training histogram ratio matrices M. This prediction is achieved by inputting the first bin-ratio vector 302 into a first artificial neural network (ANN) model, such as the ANN model depicted in Figure 4.

The ANN architecture 400 as shown in Figure 4 comprises an input layer 402; a hidden layer 404; and an output layer 406. Each layer 402, 404, 406 comprises a plurality artificial neurons (herein referred to as "nodes") connected via edges. The nodes and edges have associated weights configured to adjust the contribution of particular input data over other input data. The output from each layer 402, 404, 406 propagates to the next layer, and a function is applied to each output. An example function is the rectified linear function.

The ANN also comprises a plurality of parameters. The plurality of parameters include: a number of neurons in the hidden layer; a learning rate; and a regularization strength.

The input layer 402 is configured to receive the bin-ratio vector. In particular, the input layer 402 comprises a plurality of input nodes 402A, 402B, 402C, each configured to receive a respective entry of a bin-ratio vector. Accordingly, there are N input nodes, wherein N is the length of the input bin-ratio vector. For example, in step 512, the input node 402A is configured to receive the first entry of the first bin-ratio vector 302 of the histogram ratio matrix 300. The input node 402B is configured to receive the second entry of the first bin-ratio vector 302 of the histogram ratio matrix 300. Finally, the input node 402C is configured to receive the 1023^{rd} (i.e. the last) entry of the first bin-ratio vector 302 of the histogram ratio matrix 300.

The hidden layer 404 is configured to apply a non-linear transformation to the inputs received via the input nodes 402A, 402B, 402C. The hidden layer 404 comprises a plurality of hidden layer nodes 404A, 404B, 404C, 404D, each being an ANN model. The hidden layer 404 may also comprise a plurality of layers.

In some examples, the hidden layer 404 may comprises a sigmoid activation layer (not shown), configured to apply a sigmoid activation function, 1/(1 + *e^{-x}*). The sigmoid activation layer may be used for cases where single isotopes are to be identified. The sigmoid activation layer may also be used for cases where there are a plurality of isotopes to be identified from a single radiation source.

The output layer 406 is configured to provide an isotope prediction associated with each of the bin-ratio vector entries, following the application of the hidden layer. In particular, the output layer 406 comprises a plurality of output nodes 406A, 406B, 406C, each configured to output a respective isotope prediction. For example, the output node 406A may output a first isotope prediction, such as ²⁴¹Am, associated with the first entry of the first bin-ratio vector 302 of the histogram ratio matrix 300. The output node 406B may output a second isotope prediction, such as ¹³⁷Cs associated with the second entry of the first bin-ratio vector 304 of the histogram ratio matrix 300. The output node 406B may output a third isotope prediction, such as ¹⁵²Eu, associated with the 1023^{rd} (i.e. final) entry of the first bin-ratio vector 306 of the histogram ratio matrix 300.

In the case of a single isotope classification, the output nodes apply a softmax function.

In the case of a multi-label problem, the output layer 406 may be configured to employ a sigmoid activation function, 1/(1 *+ e^{-x}*)*,* wherein x is a weighted sum of inputs that it is passed through a sigmoid activation function which then serves as an input to the next layer. Accordingly, by applying the sigmoid activation function, the output layer 406 may restrict the output to a value between 0 and 1. In this case, a threshold may be implemented wherein if the output layer provides a value greater than the threshold, a particular isotope may be identified. For example, if threshold is 0.5, an output layer value greater than 0.5 may indicate that the classification associated with the output layer value is to be output.

Turning back to the method 200, at step 216, a cost function is evaluated for the first set of isotope predictions.

In the case of a single isotope classification, the cost function is a cross-entropy loss function: $E = - \frac{1}{K} {\sum }_{i = 1}^{k} {t}_{i} log \left(\hat{{t}_{ι}}\right) + \left(1-{t}_{i}\right) log \left(1-\hat{{t}_{ι}}\right)$

Wherein K is the total number of output nodes 406, *tᵢ* is the correct isotope classification, and $\hat{{t}_{ι}}$ is the predicted classification provided by the respective output nodes 406. In the present case, K is 1023 for the first bin-ratio vector.

In the case of multiple isotope classification, the cost function is a mean squared error function having L₂ regularization to penalize network weights of higher values and prevent overfitting: $E = \frac{1}{K} {\sum }_{i = 1}^{k} \left({t}_{i}-\hat{{t}_{ι}}\right) + \frac{1}{2} {\sum }_{j} {λw}_{j}^{2}$

Wherein *wⱼ* is a weight and *λ* is a regularization strength, *tᵢ* is the correct isotope classification, and $\hat{{t}_{ι}}$ is the predicted classification provided by the respective output nodes 406.

At step 218, a second set of isotope prediction is determined for the second bin-ratio vector 304 of each of the training histogram ratio matrices M. This prediction is achieved in the same way as the prediction of step 512, except by using a second ANN model. In this instance, since the length of the second bin-ratio vector 304 is 1022, the second ANN model comprises 1022 input nodes and output nodes. The parameters of the second ANN model are configured to minimise the cost function determined in step 514. Accordingly, a new ANN model is added to the ANN ensemble, containing the second bin-ratio vectors associated with each of plurality of known isotopes.

Steps 216 and 218 are repeated, with the output of step 218 being used for the evaluation of the cost function in step 216 of the subsequent iteration. Additionally, each subsequent iteration of step 516 utilises the subsequent bin-ratio vector of each of the plurality of known isotopes.

Steps 216 and 218 may be repeated until a threshold is reached. The threshold may be an output difference threshold. For example, when the difference between the predicted classification $\hat{{t}_{ι}}$ and the correct isotope classification is less than or equal to the output difference threshold, the method 500 may finish. An example output difference threshold may be 5 × 10⁻². The threshold may provide an indication as to whether the ANN model meets a performance metric.

Accordingly, each subsequent ANN is trained through error backpropagation to minimize the cost function with respect to the node weights. A scaled conjugate gradient (SCG) method maybe utilised for faster convergence.

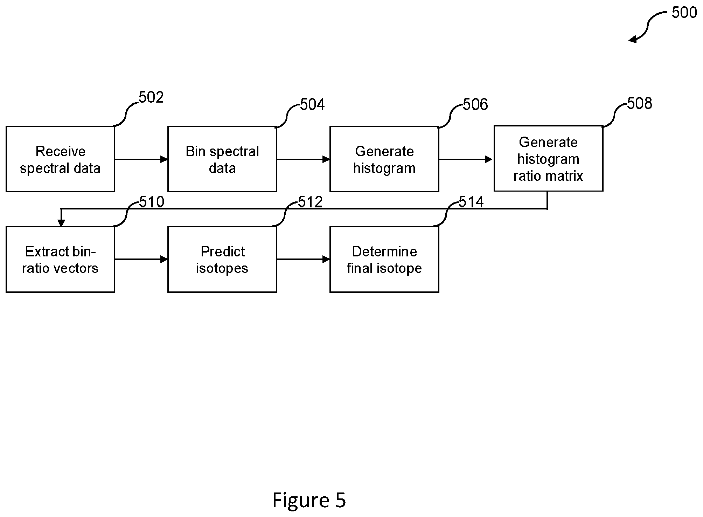

Turning now to Figure 5, there is depicted a method 200 for identifying isotopes using the system 100 of Figure 1.

In a first step 502, the computing device 102 collects or receives spectral data from the spectrometer 104. The spectral data is energy associated with gamma rays received and measured by the spectrometer 104. Accordingly, a gamma ray spectrum may be generated.

Optionally, the spectral data may be filtered to match a characteristic resolution of the spectrometer 104, in order to reduce artificial features generated by statistical noise.

At step 504, the spectral data is binned into n spectral bins. That is, each of the spectral data are grouped according to their energy. For example, for n = 1024, 1024 bins may be selected, each corresponding to a particular energy range. For a measured energy range of 10⁴ to 10⁷ eV, each bin may span a range of roughly 10³ eV. Accordingly, each of the spectral data are allocated to one of the 1024 bins corresponding to the measured energy.

At step 506, a histogram is generated based on the n spectral bins. The histogram comprises n entries and provides an indication of how many of the spectral data fall within each bin. In other words, the histogram provides a bin count associated with each bin. In the present example, the histogram comprises 1024 entries.

At step 508, a histogram ratio matrix M is generated. The histogram ratio matrix M is a square matrix of length n. In the present example, the histogram ratio matrix M is a square matrix of length 1024. The entries M_{i,j} of the histogram ratio matrix M are the bin count of the i^{th} bin divided by the bin count of the j^{th} bin: $M = {\left[\frac{{h}_{i}}{{h}_{j}}\right]}_{1 \leq i , j \leq n} = \left[\begin{matrix}\frac{{h}_{1}}{{h}_{1}} & \frac{{h}_{2}}{{h}_{1}} & ⋯ & \frac{{h}_{n}}{{h}_{1}} \\ \frac{{h}_{1}}{{h}_{2}} & \frac{{h}_{2}}{{h}_{2}} & ⋯ & \frac{{h}_{n}}{{h}_{2}} \\ ⋮ & ⋮ & ⋱ & ⋮ \\ \frac{{h}_{1}}{{h}_{n}} & \frac{{h}_{2}}{{h}_{n}} & … & \frac{{h}_{n}}{{h}_{n}}\end{matrix}\right]$

The skilled addressee will appreciate that the ratios are reciprocal and as such, the upper and lower triangles of the matrix are identical and the matrix diagonal is constant.

At step 510, a plurality of bin-ratio vectors are extracted from the histogram ratio matrix M. This extraction step can be more easily understood with reference to Figure 3, which depicts a histogram ratio matrix 300 of a 1024 bin histogram.

For a histogram ratio matrix M of n bins, the number of bin-ratio vectors is n-1. Accordingly, the histogram ratio matrix M of the present example having 1024 bins, has 1023 bin-ratio vectors. The entries of the y^{th} bin-ratio vector are a bin-ratio of a first bin and a second bin, wherein the second bin is separated from the first bin by y bins. In particular, a first bin-ratio vector 302 (having a length of 1023) contains ratios between the bin counts of adjacent bins (i.e. separated by 1 bin). A second bin-ratio vector 304 (having a length of 1022) contains ratios between bin counts of second neighbours. A final (i.e. the 1023^{rd}) bin-ratio vector 306 contains a ratio between the last (i.e. 1024^{th} bin count) and the first bin count, due to the separation of 1023 bins. Advantageously, feature diversity is introduced through ratios measured at multiple distance scales between the bins, thereby improving the robustness of the present method.

The bin-ratio vectors may each be stored in a respective dataset.

At step 512, an isotope prediction is determined for each of the bin-ratio vectors. In the present example, an artificial neural network (ANN) model, as depicted in Figure 4, is used for each of the bin-ratio vectors. The isotope prediction may be in the form of an isotope probability such that the ANN model provides a probability of the radioactive source being a particular isotope. Accordingly, for the present example, an isotope prediction associated with each bin-ratio vector of the histogram ratio matrix 300 is generated by the method 200. Further detail regarding the ANN model and architecture is discussed in relation to Figures 4 and 5.

In a final step 514 of the method 200, a final isotope prediction is determined. The final isotope prediction is determined through an ensemble forecasting step. In particular, the isotope predictions associated with each of the bin-ratio vectors, as determined in step 512 of the method 500, are combined. In the present example, the output of step 514 is the majority output of the isotope predictions associated with each of the bin-ratio vectors. That is, the isotope having the most isotope predictions or the highest cumulative probability will be output by step 514.

In the present example, the isotope ⁵⁷Co was output by 879 output nodes of the output layer 406. Accordingly, the final isotope prediction is ⁵⁷Co.

The description provided herein may be directed to specific implementations. It should be understood that the discussion provided herein is provided for the purpose of enabling a person with ordinary skill in the art to make and use any subject matter defined herein by the subject matter of the claims.

It should be intended that the subject matter of the claims not be limited to the implementations and illustrations provided herein, but include modified forms of those implementations including portions of implementations and combinations of elements of different implementations in accordance with the claims.

Reference has been made in detail to various implementations, examples of which are illustrated in the accompanying drawings and figures. In the detailed description, numerous specific details are set forth to provide a thorough understanding of the invention provided herein. However, the invention provided herein may be practiced without these specific details. In some other instances, well-known methods, procedures, components, circuits, and networks have not been described in detail so as not to unnecessarily obscure details of the embodiments.

It should also be understood that, although the terms first, second, etc. may be used herein to describe various elements, these elements should not be limited by these terms. These terms are only used to distinguish one element from another. For example, a first element could be termed a second element, and, similarly, a second element could be termed a first element. The first element and the second element are both elements, respectively, but they are not to be considered the same element.

The terminology used in the description of the invention provided herein is for the purpose of describing particular implementations and is not intended to limit the invention provided herein. As used in the description of the invention provided herein and appended claims, the singular forms "a," "an," and "the" are intended to include the plural forms as well, unless the context clearly indicates otherwise. The term "and/or" as used herein refers to and encompasses any and all possible combinations of one or more of the associated listed items. The terms "includes," "including," "comprises," and/or "comprising," when used in this specification, specify a presence of stated features, integers, steps, operations, elements, and/or components, but do not preclude the presence or addition of one or more other features, integers, steps, operations, elements, components and/or groups thereof.

The scope of the invention is defined by the appended claims.

## Claims

1. An isotope identification method performed by a computing device, comprising the steps of:
collecting, from a spectroscopy device, a plurality of spectral data;
generating, by a processor of the computing device, a plurality of datasets based on the plurality of spectral data;
determining, by the processor, a plurality of isotope probabilities, each isotope probability of the plurality of isotope probabilities corresponding to a respective dataset of the plurality of datasets;
wherein the isotope probability is indicative of a probability that the corresponding dataset of the plurality of datasets corresponds to a particular isotope; and
identifying, by the processor, an isotope based on the plurality of isotope probabilities;
wherein the isotope probability is determined by using an ensemble; said ensemble comprising a plurality of classifications generated by a multiclass classifier, each classification corresponding to a respective dataset; and
**characterized in that**
the ensemble is an artificial neural network (ANN) ensemble; said ANN ensemble comprising a plurality of ANN classifications, each generated by an ANN, each ANN classification corresponding to a respective dataset.

2. The method of claim 1, wherein each dataset of the plurality of datasets comprises binned data, said dataset being representative of a functional relationship between a first bin of the binned data and a second bin of the binned data.

3. The method of claim 2, wherein the plurality of datasets each comprise a respective bin-ratio vector.

4. The method of claim 3, wherein generating the plurality of datasets comprises:
binning, by the processor, the plurality of spectral data into n spectral bins;
generating, by the processor, a plurality of bin-ratio vectors; and
storing, by the processor, in each dataset of the plurality of datasets, a respective bin-ratio vector.

5. The method of claim 4, wherein generating the plurality of bin-ratio vectors comprises:
generating, by the processor, a histogram based on the n spectral bins;
generating, by the processor, a histogram ratio matrix M based on the histogram;
wherein the histogram ratio matrix M is a square matrix of length n; and
extracting, by the processor, the plurality of bin-ratio vectors from the histogram ratio matrix;
wherein there are k bin-ratio vectors and k = n-1.

6. The method of claim 5, wherein each entry M_{i,j} of the histogram ratio matrix M is the i^{th} bin divided by the j^{th} bin; preferably wherein the entries of the y^{th} bin-ratio vector are a bin-ratio of a first bin and a second bin, wherein the second bin is separated from the first bin by y bins.

7. The method of claim 2, wherein the bin data comprises a bin difference.

8. The method of any one of the preceding claims, wherein the isotope probability is determined based on the plurality of **ANN** classifications.

9. The method of any of the preceding claims, wherein each ANN comprises one or more parameters, the parameters being one or more selected from the range of:
a hidden layer comprising a number of neurons;
a learning rate; and
a regularization strength;
preferably wherein the parameters are selected by applying a random search method.

10. The method of any one of the preceding claims, wherein the multiclass classifier is optimised; preferably wherein the multiclass classifier is optimised through error backpropagation, the multiclass classifier being configured to minimize a cost function with respect to a network weight.

11. The method of claim 10, wherein the cost function is a cross-entropy loss function for single isotope classification; and/or wherein the cost function is a mean-squared error cost function for isotope mixture classification.

12. The method of claim 10 or 11, wherein the cost function comprises a penalty term, said penalty term being an L2 regularization element.

13. The method of claim 11 or 12, wherein the multiclass classifier is optimised according to a scaled conjugate gradient method.

14. The method of any one of claims 11 to 13, wherein the multiclass classifier is trained until a threshold difference between a predicted output and a correct output reaches a threshold.

15. The method of any preceding claim, wherein the isotope is identified by an ensemble forecast; preferably wherein the ensemble forecast comprises:
determining the isotope having the highest probability for each dataset of the plurality of datasets; and
identifying the isotope having the highest number of highest probabilities.

## Patentansprüche

1. Ein Isotopenidentifizierungsverfahren, das durch eine Rechenvorrichtung durchgeführt wird, das die folgenden Schritte beinhaltet:
Sammeln, von einer Spektroskopievorrichtung, einer Vielzahl von Spektraldaten;
Erzeugen, durch einen Prozessor der Rechenvorrichtung, einer Vielzahl von Datensätzen basierend auf der Vielzahl von Spektraldaten;
Bestimmen, durch den Prozessor, einer Vielzahl von Isotopenwahrscheinlichkeiten,
wobei jede Isotopenwahrscheinlichkeit der Vielzahl von Isotopenwahrscheinlichkeiten einem jeweiligen Datensatz der Vielzahl von Datensätzen entspricht;
wobei die Isotopenwahrscheinlichkeit eine Wahrscheinlichkeit angibt, dass der entsprechende Datensatz der Vielzahl von Datensätzen einem speziellen Isotop entspricht; und
Identifizieren, durch den Prozessor, eines Isotops basierend auf der Vielzahl von Isotopenwahrscheinlichkeiten;
wobei die Isotopenwahrscheinlichkeit unter Verwendung eines Ensembles bestimmt wird; wobei das Ensemble eine Vielzahl von Klassifikationen beinhaltet, die durch einen Mehrklassenklassifikator erzeugt werden, wobei jede Klassifikation einem jeweiligen Datensatz entspricht; und
**dadurch gekennzeichnet, dass**
das Ensemble ein Ensemble künstlicher neuronaler Netzwerke (KNN-Ensemble) ist;
wobei das KNN-Ensemble eine Vielzahl von KNN-Klassifikationen beinhaltet, die jeweils durch ein KNN erzeugt werden, wobei jede KNN-Klassifikation einem jeweiligen Datensatz entspricht.

2. Verfahren gemäß Anspruch 1, wobei jeder Datensatz der Vielzahl von Datensätzen gebinnte Daten beinhaltet, wobei der Datensatz für eine funktionale Beziehung zwischen einem ersten Bin der gebinnten Daten und einem zweiten Bin der gebinnten Daten repräsentativ ist.

3. Verfahren gemäß Anspruch 2, wobei die Vielzahl von Datensätzen jeweils einen jeweiligen Bin-Verhältnis-Vektor beinhaltet.

4. Verfahren gemäß Anspruch 3, wobei das Erzeugen der Vielzahl von Datensätzen Folgendes beinhaltet:
Binnen, durch den Prozessor, der Vielzahl von Spektraldaten in n Spektral-Bins;
Erzeugen, durch den Prozessor, einer Vielzahl von Bin-Verhältnis-Vektoren; und
Speichern, durch den Prozessor, in jedem Datensatz der Vielzahl von Datensätzen,
eines jeweiligen Bin-Verhältnis-Vektors.

5. Verfahren gemäß Anspruch 4, wobei das Erzeugen der Vielzahl von Bin-Verhältnis-Vektoren Folgendes beinhaltet:
Erzeugen, durch den Prozessor, eines Histogramms basierend auf den n Spektral-Bins;
Erzeugen, durch den Prozessor, einer Histogramm-Verhältnis-Matrix M basierend auf dem Histogramm;
wobei die Histogramm-Verhältnis-Matrix M eine quadratische Matrix der Länge n ist; und
Extrahieren, durch den Prozessor, der Vielzahl von Bin-Verhältnis-Vektoren aus der Histogramm-Verhältnis-Matrix;
wobei es k Bin-Verhältnis-Vektoren gibt und k = n - 1.

6. Verfahren gemäß Anspruch 5, wobei jeder Eintrag M_{i,j} der Histogramm-Verhältnis-Matrix M der i-te Bin geteilt durch den j-ten Bin ist; wobei vorzugsweise die Einträge des y-ten Bin-Verhältnis-Vektors ein Bin-Verhältnis eines ersten Bins und eines zweiten Bins sind, wobei der zweite Bin von dem ersten Bin durch y Bins getrennt ist.

7. Verfahren gemäß Anspruch 2, wobei die Bin-Daten eine Bin-Differenz beinhalten.

8. Verfahren gemäß einem der vorhergehenden Ansprüche, wobei die Isotopenwahrscheinlichkeit basierend auf der Vielzahl von KNN-Klassifikationen bestimmt wird.

9. Verfahren gemäß einem der vorhergehenden Ansprüche, wobei jedes KNN einen oder mehrere Parameter beinhaltet, wobei die Parameter einer oder mehrere sind, ausgewählt aus dem Bereich von:
einer verborgenen Schicht, die eine Anzahl von Neuronen beinhaltet;
einer Lernrate; und
einer Regularisierungsstärke;
wobei vorzugsweise die Parameter durch Anwenden eines Zufallssuchverfahrens ausgewählt werden.

10. Verfahren gemäß einem der vorhergehenden Ansprüche, wobei der Mehrklassenklassifikator optimiert wird; wobei vorzugsweise der Mehrklassenklassifikator durch Fehlerrückpropagierung optimiert wird, wobei der Mehrklassenklassifikator konfiguriert ist, um eine Kostenfunktion in Bezug auf ein Netzwerkgewicht zu minimieren.

11. Verfahren gemäß Anspruch 10, wobei die Kostenfunktion eine Kreuzentropie-Verlustfunktion für eine Einzelisotopenklassifikation ist; und/oder wobei die Kostenfunktion eine Kostenfunktion des mittleren quadratischen Fehlers für eine Isotopengemischklassifikation ist.

12. Verfahren gemäß Anspruch 10 oder 11, wobei die Kostenfunktion einen Strafterm beinhaltet, wobei der Strafterm ein L2-Regularisierungselement ist.

13. Verfahren gemäß Anspruch 11 oder 12, wobei der Mehrklassenklassifikator nach einem skalierten Verfahren der konjugierten Gradienten optimiert wird.

14. Verfahren gemäß einem der Ansprüche 11 bis 13, wobei der Mehrklassenklassifikator trainiert wird, bis eine Schwellenwertdifferenz zwischen einer vorhergesagten Ausgabe und einer korrekten Ausgabe einen Schwellenwert erreicht.

15. Verfahren gemäß einem der vorhergehenden Ansprüche, wobei das Isotop durch eine Ensemble-Vorhersage identifiziert wird; wobei vorzugsweise die Ensemble-Vorhersage Folgendes beinhaltet:
Bestimmen, für jeden Datensatz der Vielzahl von Datensätzen, des Isotops, das die höchste Wahrscheinlichkeit aufweist; und
Identifizieren des Isotops, das die höchste Anzahl von höchsten Wahrscheinlichkeiten aufweist.

## Revendications

1. Un procédé d'identification d'isotope réalisé par un dispositif informatique, comprenant les étapes :
de recueil, à partir d'un dispositif de spectroscopie, d'une pluralité de données spectrales ;
de génération, par un processeur du dispositif informatique, d'une pluralité d'ensembles de données sur la base de la pluralité de données spectrales ;
de détermination, par le processeur, d'une pluralité de probabilités d'isotope, chaque probabilité d'isotope de la pluralité de probabilités d'isotope correspondant à un ensemble de données respectif de la pluralité d'ensembles de données ;
dans lequel la probabilité d'isotope est indicative d'une probabilité que l'ensemble de données correspondant de la pluralité d'ensembles de données corresponde à un isotope particulier ; et
d'identification, par le processeur, d'un isotope sur la base de la pluralité de probabilités d'isotope ;
dans lequel la probabilité d'isotope est déterminée en utilisant une collection ; ladite collection comprenant une pluralité de classifications générées par un classificateur multiclasse, chaque classification correspondant à un ensemble de données respectif ; et
**caractérisé en ce que**
la collection est une collection de réseaux de neurones artificiels (RNA) ; ladite collection de RNA comprenant une pluralité de classifications de RNA, chacune étant générée par un RNA, chaque classification de RNA correspondant à un ensemble de données respectif.

2. Le procédé de la revendication 1, dans lequel chaque ensemble de données de la pluralité d'ensembles de données comprend des données compartimentées, ledit ensemble de données étant représentatif d'une relation fonctionnelle entre un premier compartiment des données compartimentées et un deuxième compartiment des données compartimentées.

3. Le procédé de la revendication 2, dans lequel la pluralité d'ensembles de données comprennent chacun un vecteur de rapport de compartiments respectif.

4. Le procédé de la revendication 3, dans lequel la génération de la pluralité d'ensembles de données comprend :
le compartimentage, par le processeur, de la pluralité de données spectrales en n compartiments spectraux ;
la génération, par le processeur, d'une pluralité de vecteurs de rapport de compartiments ; et
le stockage, par le processeur, dans chaque ensemble de données de la pluralité d'ensembles de données, d'un vecteur de rapport de compartiments respectif.

5. Le procédé de la revendication 4, dans lequel la génération de la pluralité de vecteurs de rapport de compartiments comprend :
la génération, par le processeur, d'un histogramme sur la base des n compartiments spectraux ;
la génération, par le processeur, d'une matrice M de rapport d'histogramme sur la base de l'histogramme ;
dans lequel la matrice M de rapport d'histogramme est une matrice carrée de longueur n ; et
l'extraction, par le processeur, de la pluralité de vecteurs de rapport de compartiments à partir de la matrice de rapport d'histogramme ;
dans lequel il y a k vecteurs de rapport de compartiments et k = n - 1.

6. Le procédé de la revendication 5, dans lequel chaque entrée M_{i,j} de la matrice M de rapport d'histogramme est le i^{ème} compartiment divisé par le j^{ième} compartiment ; de préférence dans lequel les entrées du y^{ième} vecteur de rapport de compartiments sont un rapport de compartiments d'un premier compartiment et d'un deuxième compartiment, le deuxième compartiment étant séparé du premier compartiment de y compartiments.

7. Le procédé de la revendication 2, dans lequel les données de compartiment comprennent une différence de compartiments.

8. Le procédé de l'une quelconque des revendications précédentes, dans lequel la probabilité d'isotope est déterminée sur la base de la pluralité de classifications de RNA.

9. Le procédé de n'importe lesquelles des revendications précédentes, dans lequel chaque RNA comprend un ou plusieurs paramètres, les paramètres étant un ou plusieurs paramètres sélectionnés dans la gamme :
une couche cachée comprenant un certain nombre de neurones ;
un taux d'apprentissage ; et
une force de régularisation ;
de préférence dans lequel les paramètres sont sélectionnés en appliquant un procédé de recherche aléatoire.

10. Le procédé de l'une quelconque des revendications précédentes, dans lequel le classificateur multiclasse est optimisé ; de préférence dans lequel le classificateur multiclasse est optimisé par rétropropagation d'erreur, le classificateur multiclasse étant configuré pour minimiser une fonction de coût par rapport à un poids de réseau.

11. Le procédé de la revendication 10, dans lequel la fonction de coût est une fonction de perte d'entropie croisée pour une classification d'isotope unique ; et/ou dans lequel la fonction de coût est une fonction de coût d'erreur quadratique moyenne pour une classification de mélange d'isotopes.

12. Le procédé de la revendication 10 ou de la revendication 11, dans lequel la fonction de coût comprend un terme de pénalité, ledit terme de pénalité étant un élément de régularisation L2.

13. Le procédé de la revendication 11 ou de la revendication 12, dans lequel le classificateur multiclasse est optimisé selon un procédé de gradient conjugué mis à l'échelle.

14. Le procédé de l'une quelconque des revendications 11 à 13, dans lequel le classificateur multiclasse est entraîné jusqu'à ce qu'une différence seuil entre une sortie prédite et une sortie correcte atteigne un seuil.

15. Le procédé de n'importe quelle revendication précédente, dans lequel l'isotope est identifié par une prévision de collection ; de préférence dans lequel la prévision de collection comprend :
la détermination de l'isotope ayant la probabilité la plus élevée pour chaque ensemble de données de la pluralité d'ensembles de données ; et
l'identification de l'isotope ayant le nombre le plus élevé de probabilités les plus élevées.
